# EUROPEAN PATENT APPLICATION

(11) **EP 3 079 189 A1**
(43) Date of publication of application: **12.10.2016**
(21) Application number: 14876837.7
(22) Date of filing: 15.07.2014
(51) Int. Cl.: H01M 4/60, H01M 4/137, H01M 10/052, C01B 31/04

(54) **QUINONE COMPOUND-GRAPHENE COMPOSITE, PREPARATION METHOD THEREFOR AND FLEXIBLE LITHIUM SECONDARY BATTERY**

(30) Priority: 31.12.2013 CN 201310752362
(71) Applicant: Huawei Technologies Co., Ltd., Longgang District Shenzhen, Guangdong 518129 (CN)
(72) Inventor: WANG, Pinghua, Shenzhen Guangdong 518129 (CN)
(74) Representative: Pfenning, Meinig & Partner mbB
(86) International application number: PCT/CN2014/082200
(87) International publication number: WO 2015/101010

(57) **Abstract**

The present invention provides a quinone compound-graphene composite material, including quinone compound and graphene, where the quinone compound is chemically bonded on the surface of the graphene, and the quinone compound is quinone compound monomer or quinone polymer. The quinone compound-graphene composite material has high energy density, high flexibility, high conductivity, and high stability, and can be used as cathode material for preparing flexible electrode. Embodiments of the present invention further provide preparation method of quinone compound-graphene composite material, and flexible lithium secondary battery using the quinone compound-graphene composite material as cathode active material.

## Description

### CROSS-REFERENCE TO RELATED APPLICATION

This application claims priority to Chinese Patent Application No. 201310752362.1, filed with the Chinese Patent Office on December 31, 2013 and entitled "QUINONE COMPOUND-GRAPHENE COMPOSITE MATERIAL, PREPARATION METHOD THEREOF, AND FLEXIBLE LITHIUM SECONDARY BATTERY", which is incorporated herein by reference in its entirety.

### TECHNICAL FIELD

The present invention relates to the field of energy materials, and in particular, to a quinone compound-graphene composite material, a preparation method thereof, and a flexible lithium secondary battery.

### BACKGROUND

As people's demands for miniaturization of electronic products are growing, flexible, wearable, and portable electronic products, such as electronic paper, folding display screens, electronic labels, and flexible sensors, has become a future development trend. A flexible energy storage device is formed by flexible electrode, electrolyte, flexible packaging material, and the like, where development of the flexible electrode is a key to the development of the flexible energy storage device.

A compound or polymer is also applicable to preparing electrode materials of the flexible energy storage device. A compound or organic polymer is generally used as organic cathode material of organic lithium ion battery. Organic conductive polymer and composite material thereof (such as polyaniline, polythiophene, polypyrrole, and polyacetylene), organic sulfide or polysulfide (such as 2,5-dimercapto-thiadiazole, carbyne polysulfide, and polyaniline polysulfide), and oxygenated functional group compound (such as quinones and the polymer thereof, and carbonyl compound with anhydride as representative) are mainly used as organic cathode materials.

The foregoing organic cathode materials have advantages of high theoretical capacity (400 to 900 mAh/g), cost-effective synthesis, recyclability, mechanical flexibility, and designable molecular height. However, the organic cathode materials generally have low conductivity, poor performance stability as which have to need high electrolyte absorbing and easy to dissolve in electrolyte, and then affected the battery performance of cycle life and power ability which is far poorer than those of a conventional lithium ion battery.

### SUMMARY

In view of this, a first aspect of embodiments of the present invention provides a quinone compound-graphene composite material. The composite material has high energy density, high flexibility, high conductivity, and high stability, which solves the problem in the prior art that an organic cathode material has poor conductivity and poor stability. The composite material can be used as cathode material for preparing flexible battery.

According to the first aspect, the embodiments of the present invention provide a quinone compound-graphene composite material, including quinone compound and graphene, where the quinone compound is chemically bonded on the surface of the graphene, and the quinone compound is quinone compound monomer or quinone polymer.

The quinone compound-graphene composite material provided by the embodiments of the present invention has high energy density, high flexibility, high conductivity, and high stability. The quinone compound has relatively high specific capacity and redox potential; double carbonyl in quinone compound has electrochemical activity point, oxygen atom on each carbonyl obtains one electron during discharging, and lithium ion is intercalated to produce lithium enolate; during charging, lithium ion is deintercalated, carbonyl functional groups are reduced, and lithium ion is intercalated and deintercalated through conversion between structures of carbonyls and enol; the quinone compound exhibits good structural stability in a process of electrochemical reaction,, which contributes to good cycle stability of the quinone compound. The graphene has excellent conductivity and flexibility, and can greatly improve conductivity performance and flexibility of the composite material, so that the composite material is applicable to use as cathode material for preparing flexible battery, and has high capacity.

Preferably, the surface of the graphene contains carboxyl functional group, the quinone compound contains amino functional group, and the quinone compound is bonded on the surface of the graphene by amido bond formed by the carboxyl functional group and the amino functional group.

Preferably, the quinone compound monomer is any one of benzoquinone, naphthoquinone, anthraquinone, and phenanthraquinone, or the isomer of any one of benzoquinone, naphthoquinone, anthraquinone, and phenanthraquinone.

Preferably, the quinone polymer is polymer formed by any one or more than one of benzoquinone, naphthoquinone, anthraquinone, and phenanthraquinone, or the polymer formed by the isomer of any one of benzoquinone, naphthoquinone, anthraquinone, and phenanthraquinone or formed by the isomers of more than one of benzoquinone, naphthoquinone, anthraquinone, and phenanthraquinone.

Preferably, the quinone compound accounts for 20 to 80% by weight of the quinone compound-graphene composite material.

In the quinone compound-graphene composite material provided by the first aspect of the embodiments of the present invention, high conductivity graphene and the quinone compound are chemical compounded. The composite material has high energy density, high flexibility, high conductivity, and high stability, which solves the problem in the prior art that the organic cathode material has poor conductivity and poor stability. The composite material can be used as cathode for preparing flexible battery.

According to second aspect, the embodiments of the present invention provide a preparation method of quinone compound-graphene composite material, including the following steps:
aminating quinone compound monomer by nitration reduction method, to obtain mixed liquor containing the aminated quinone compound monomer;
filtering, washing, and drying the mixed liquor containing the aminated quinone compound monomer, to obtain aminated quinone compound monomer powder;
adding graphene oxide powder to mixed acid that contains concentrated sulfuric acid and concentrated nitric acid in preset proportion, to react for 5 to 15 h at 50 to 60 °C, and then performing operations of filtration, washing, and vacuum drying, to obtain surface-carboxylated graphene powder;
adding the aminated quinone compound monomer powder and the surface-carboxylated graphene powder to water or ethyl alcohol, and performing ultrasonic dispersion to obtain mixed dispersed liquor; and
performing reflux reaction on the mixed dispersed liquor for 48 to 72 h at 100 to 120 °C under protection of nitrogen gas, and after reaction is finished, performing filtration, washing, and drying on the product, to obtain quinone compound monomer-graphene composite material.

Preferably, the quinone compound monomer is any one of benzoquinone, naphthoquinone, anthraquinone, and phenanthraquinone, or the isomer of any one of benzoquinone, naphthoquinone, anthraquinone, and phenanthraquinone.

Preferably, the reaction for 5 to 15 h at 50 to 60 °C is performed under conditions of stirring and ultrasonic dispersion.

Preferably, the preparation method of quinone compound-graphene composite material further includes the following steps:
weighing out the quinone compound monomer-graphene composite material, perchloric acid, saturated water solution of hydrogen peroxide, and potassium dichromate according to molar ratio of 1:2:2:2, and adding them to acetonitrile solvent, to obtain mixed solution;
adding saturated water solution of potassium dichromate dropwise to the mixed solution to react for 48 to 72 h at 30 to 50 °C under protection of argon gas, to obtain mixed liquor containing quinone polymer-graphene composite material; and
performing decompression filtration, washing, and vacuum drying on the mixed liquor containing the quinone polymer-graphene composite material, to obtain powder of the quinone polymer-graphene composite material.

The quinone compound-graphene composite material preparation method provided by the second aspect of the embodiments of the present invention has simple process, and is easy to implement in industrialized production.

According to third aspect, the embodiments of the present invention provide a preparation method of quinone compound-graphene composite material, including the following steps:
aminating quinone compound monomer by nitration reduction method, to obtain mixed liquor containing the aminated quinone compound monomer;
filtering, washing, and drying the mixed liquor containing the aminated quinone compound monomer, to obtain aminated quinone compound monomer powder;
adding the aminated quinone compound monomer powder to electrolyte of an organic propylene carbonate solvent, and polymerizing the aminated quinone compound monomer by constant potential polymerization method of three-electrode system, to obtain animated quinone polymer;
adding graphene oxide powder to mixed acid that contains concentrated sulfuric acid and concentrated nitric acid in preset proportion, to react for 5 to 15 h at 50 to 60 °C, and then performing operations of filtration, washing, and vacuum drying, to obtain surface-carboxylated graphene powder;
adding the aminated quinone polymer and the carboxylated graphene powder to water or ethyl alcohol, and performing ultrasonic dispersion to obtain mixed dispersed liquor; and
performing reflux reaction on the mixed dispersed liquor for 48 to 72 h at 100 to 120 °C under protection of nitrogen gas, and after reaction is finished, performing filtration, washing, and drying on the product, to obtain quinone polymer-graphene composite material.

Preferably, the quinone compound monomer is any one of benzoquinone, naphthoquinone, anthraquinone, and phenanthraquinone, or the isomer of any one of benzoquinone, naphthoquinone, anthraquinone, and phenanthraquinone.

Preferably, the reaction for 5 to 15 h at 50 to 60 °C is performed under conditions of stirring and ultrasonic dispersion.

The preparation method of quinone compound-graphene composite material provided by the third aspect of the embodiments of the present invention has simple process, and is easy to implement in industrialized production.

According to fourth aspect, the embodiments of the present invention provide a flexible lithium secondary battery, including flexible battery housing; flexible cathode, flexible anode cathode, and separator that are located inside the flexible battery housing, and electrolyte injected inside the flexible battery housing, where the separator is disposed between the flexible cathode and the flexible anode, the flexible cathode includes cathode active material which including a quinine-compound graphene composite material. The quinone compound-graphene composite material includes quinone compound and graphene, where the quinone compound is chemically bonded on the surface of the graphene, and the quinone compound is quinone compound monomer or quinone polymer.

Preferably, the flexible anode includes anode active material, and the anode active material is graphene, carbon nanotube, silicon film, or tin film.

The flexible lithium secondary battery provided by the fourth aspect of the embodiments of the present invention exhibits high flexibility, high rate performance, high energy density, and high stability, and is applicable to future flexible electronic device.

In conclusion, in the quinone compound-graphene composite material provided by the first aspect of the embodiments of the present invention, high conductivity graphene and the quinone compound are chemical compounded. The composite material has high energy density, high flexibility, high conductivity, and high stability, which solves the problem in the prior art that organic cathode material has poor conductivity and poor stability. The composite material can be used as cathode material for preparing flexible battery. The preparation methods of quinone compound-graphene composite material provided by the second and third aspects of the embodiments of the present invention have simple process, and are easy to implement in industrialized production. The flexible lithium secondary battery provided by the fourth aspect of the embodiments of the present invention exhibits high flexibility, high rate performance, high energy density, and high stability, and is applicable to future flexible electronic device.

Advantages of the embodiments of the present invention are partially described below in this specification, and part of these advantages are apparent according to this specification, or may be known by practicing the embodiments of the present invention.

### BRIEF DESCRIPTION OF DRAWINGS

FIG. 1 is flowchart of preparing p-benzoquinone-graphene composite material according to Embodiment 1 of the present invention;
FIG. 2 is flowchart of preparing polyaminated anthraquinone-graphene composite material according to Embodiment 2 of the present invention;
FIG. 3 is flowchart of preparing polyaminated anthraquinone-graphene composite material according to Embodiment 3 of the present invention; and
FIG. 4 is schematic structural diagram of flexible lithium secondary battery according to an embodiment of the present invention.

### DESCRIPTION OF EMBODIMENTS

The following descriptions are exemplary implementations of embodiments of the present invention. It should be noted that a person of ordinary skill in the art may make several improvements and polishing without departing from the principle of the embodiments of the present invention and the improvements and polishing shall fall within the protection scope of the embodiments of the present invention.

The following further describes in detail the embodiments of the present invention by multiple embodiments. The embodiments of the present invention are not limited to the following specific embodiments. Implementations can be modified within the scope of the claims to a certain extent.

A first aspect of the embodiments of the present invention provides a quinone compound-graphene composite material. The composite material has high energy density, high flexibility, high conductivity, and high stability, which solves the problem in the prior art that organic cathode material has poor conductivity and poor stability. The composite material can be used as cathode material for preparing flexible battery.

According to the first aspect, the embodiments of the present invention provide a quinone compound-graphene composite material, including quinone compound and graphene, where the quinone compound is chemically bonded on the surface of the graphene, and the quinone compound is quinone compound monomer or quinone polymer.

The quinone compound-graphene composite material provided by the embodiments of the present invention has high energy density, high flexibility, high conductivity, and high stability. The quinone compound has relatively high specific capacity and redox potential; double carbonyl in the quinone compound has electrochemical activity point, oxygen atom on each carbonyl obtains one electron during discharging, and lithium ion is intercalated to produce lithium enolate; during charging, lithium ion is deintercalated, carbonyls are reduced, and lithium ion is intercalated and deintercalated through conversion between structures of carbonyls and enol; in process of electrochemical reaction, the quinone compound exhibits good structural stability, which contributes to good cycle stability of the quinone compound. The graphene has excellent conductivity and flexibility, and can greatly improve conductivity performance and flexibility of the composite material, so that the composite material is applicable to cathode material for preparing flexible battery, and has high capacity.

Preferably, the surface of the graphene contains carboxyl functional group, the quinone compound contains amino functional group, and the quinone compound is bonded on the surface of the graphene by amido bond formed by the carboxyl functional group and the amino functional group.

Preferably, the quinone compound monomer is any one of benzoquinone, naphthoquinone, anthraquinone, and phenanthraquinone, or the isomer of any one of benzoquinone, naphthoquinone, anthraquinone, and phenanthraquinone.

Preferably, the quinone polymer is a polymer formed by any one or more than one of benzoquinone, naphthoquinone, anthraquinone, and phenanthraquinone, or the polymer formed by the isomer of any one of benzoquinone, naphthoquinone, anthraquinone, and phenanthraquinone or formed by isomers of more than one of benzoquinone, naphthoquinone, anthraquinone, and phenanthraquinone.

Preferably, the quinone compound accounts for 20 to 80% by weight of the quinone compound-graphene composite material.

The graphene in the quinone compound-graphene composite material according to the embodiments of the present invention is graphene oxide.

In the quinone compound-graphene composite material provided by the first aspect of the embodiments of the present invention, high conductivity graphene and the quinone compound are chemical compounded. The composite material has high energy density, high flexibility, high conductivity, and high stability, which solves the problem in the prior art that organic cathode material has poor conductivity and poor stability. The composite material can be used as cathode material for preparing flexible battery.

According to second aspect, the embodiments of the present invention provide a preparation method of quinone compound-graphene composite material, including the following steps:
aminating quinone compound monomer by nitration reduction method, to obtain mixed liquor containing the aminated quinone compound monomer;
filtering, washing, and drying the mixed liquor containing the aminated quinone compound monomer, to obtain aminated quinone compound monomer powder;
adding graphene oxide powder to mixed acid that contains concentrated sulfuric acid and concentrated nitric acid in preset proportion, to react for 5 to 15 h at 50 to 60 °C, and then performing operations of filtration, washing, and vacuum drying, to obtain surface-carboxylated graphene powder;
adding the aminated quinone compound monomer powder and the surface-carboxylated graphene powder to water or ethyl alcohol, and performing ultrasonic dispersion to obtain mixed dispersed liquor; and
performing reflux reaction on the mixed dispersed liquor for 48 to 72 h at 100 to 120 °C under protection of nitrogen gas, and after reaction is finished, performing filtration, washing, and drying on the product, to obtain quinone compound monomer-graphene composite material.

Preferably, the quinone compound monomer is any one of benzoquinone, naphthoquinone, anthraquinone, and phenanthraquinone, or the isomer of any one of benzoquinone, naphthoquinone, anthraquinone, and phenanthraquinone.

Preferably, the specific operation of aminating quinone compound monomer by nitration reduction method is: aminating the quinone compound monomer by mixed acid containing concentrated sulfuric acid and concentrated nitric acid, to obtain aminated quinone compound monomer, and then performing reduction by sodium sulfide.

In the mixed acid containing concentrated sulfuric acid and concentrated nitric acid according to the embodiments of the present invention, the volume ratio of concentrated sulfuric acid to concentrated nitric acid preferably is 3:1.

Preferably, the reaction for 5 to 15 h at 50 to 60 °C is performed under conditions of stirring and ultrasonic dispersion.

Preferably, the preparation method of quinone compound-graphene composite material further includes the following steps:
weighing out the quinone compound monomer-graphene composite material, perchloric acid, saturated water solution of hydrogen peroxide, and potassium dichromate according to molar ratio of 1:2:2:2, and adding them to acetonitrile solvent, to obtain a mixed solution;
adding saturated water solution of potassium dichromate dropwise to the mixed solution to react for 48 to 72 h at 30 to 50 °C under protection of argon gas, to obtain mixed liquor containing quinone polymer-graphene composite material; and
performing decompression filtration, washing, and vacuum drying on the mixed liquor containing the quinone polymer-graphene composite material, to obtain powder of the quinone polymer-graphene composite material.

The above is polymerization reaction process of the quinone compound monomer. The polymerization reaction happens in amino connected to benzene ring and hydrogen in a para-position of the amino in the quinone compound monomer, and the amino connected to the benzene ring and the hydrogen in a para-position of the amino form the quinone polymer in head-to-tail ligation manner.

The preparation method of quinone compound-graphene composite material provided by the second aspect of the embodiments of the present invention has simple process, and is easy to implement in industrialized production.

According to third aspect, the embodiments of the present invention provide a preparation method of quinone compound-graphene composite material, including the following steps:
aminating quinone compound monomer by nitration reduction method, to obtain mixed liquor containing the aminated quinone compound monomer;
filtering, washing, and drying the mixed liquor containing the aminated quinone compound monomer, to obtain aminated quinone compound monomer powder;
adding the aminated quinone compound monomer powder to electrolyte of organic propylene carbonate solvent, and polymerizing the aminated quinone compound monomer by constant potential polymerization method of three-electrode system, to obtain animated quinone polymer;
adding graphene oxide powder to mixed acid that contains concentrated sulfuric acid and concentrated nitric acid in preset proportion, to react for 5 to 15 h at 50 to 60 °C, and then performing operations of filtration, washing, and vacuum drying, to obtain surface-carboxylated graphene powder;
adding the aminated quinone polymer and the carboxylated graphene powder to water or ethyl alcohol, and performing ultrasonic dispersion to obtain mixed dispersed liquor; and
performing reflux reaction on the mixed dispersed liquor for 48 to 72 h at 100 to 120 °C under protection of nitrogen gas, and after reaction is finished, performing filtration, washing, and drying on the product, to obtain quinone polymer-graphene composite material.

Preferably, the quinone compound monomer is any one of benzoquinone, naphthoquinone, anthraquinone, and phenanthraquinone, or the isomer of any one of benzoquinone, naphthoquinone, anthraquinone, and phenanthraquinone.

Preferably, the reaction for 5 to 15 h at 50 to 60 °C is performed under conditions of stirring and ultrasonic dispersion.

Preferably, the specific operation of aminating quinone compound monomer by nitration reduction method is: aminating the quinone compound monomer by mixed acid containing concentrated sulfuric acid and concentrated nitric acid, to obtain the aminated quinone compound monomer, and then performing reduction by sodium sulfide.

In the mixed acid containing concentrated sulfuric acid and concentrated nitric acid according to the embodiments of the present invention, the volume ratio of concentrated sulfuric acid to concentrated nitric acid preferably is 3:1.

In the process of polymerizing the aminated quinone compound monomer to obtain aminated quinone polymer, the polymerization reaction happens in amino connected to benzene ring and hydrogen in para-position of the amino in the quinone compound monomer, and the amino connected to the benzene ring and the hydrogen in para-position of the amino form the quinone polymer in head-to-tail ligation manner.

The quinone compound-graphene composite material preparation method provided by the third aspect of the embodiments of the present invention has simple process, and is easy to implement in industrialized production.

According to fourth aspect, the embodiments of the present invention provide a flexible lithium secondary battery, including flexible battery housing; flexible cathode, flexible anode, and separator that are located inside the flexible battery housing, and electrolyte injected inside the flexible battery housing, where the separator is disposed between the flexible cathode and the flexible anode, the flexible cathode includes cathode active material which including quinine-compound graphene composite material. The quinone compound-graphene composite material includes quinone compound and graphene, where the quinone compound is chemically bonded on the surface of the graphene, and the quinone compound is quinone compound monomer or quinone polymer.

Preferably, the flexible anode includes anode active material, and the anode active material is graphene, carbon nanotube, silicon film, or tin film.

Preferably, the flexible cathode includes cathode current collector, and the flexible anode includes anode current collector.

Preferably, the cathode current collector is metal foil current collector or graphene film.

Preferably, the anode current collector is metal foil current collector or graphene film. The metal foil current collector includes copper foil, aluminum foil, and the like.

The flexible cathode and the flexible anode each include binder and conductive agent. The binder is any substance that can enable an active material to adhere to particles of the conductive agent, and may be the commonly known binder, such as polyvinylidene fluoride (PVDF), polytetrafluoroethylene (PTFE), sodium carboxymethylcellulose (CMC), or styrene butadiene rubber (SBR). The conductive agent is any substance that can improve conductivity performance of the surface of an active material, and may be the commonly known conductive agent, such as conductive graphite, carbon black, carbon fiber, carbon nanotube, graphene, or metal micro-powder. The electrolyte is not specially limited, and may be the commonly known electrolyte.

Preferably, the separator is polyolefin separator, polyvinyl alcohol separator, polyvinyl chloride separator, polytetrafluoroethylene separator, polystyrene separator, polyethylene phenol separator, polymethyl methacrylate separator, polyimide separator, or polyethylene glycol terephthalate separator.

Preferably, the material of the flexible battery housing is aluminum plastic composite film.

In another exemplary implementation of the present invention, the flexible cathode includes quinone compound-graphene composite material and the flexible lithium secondary battery comprises together with the flexible cathode, the separator, the electrolyte, and the flexible battery housing.

The flexible lithium secondary battery provided by the fourth aspect of the embodiments of the present invention exhibits high flexibility, high rate performance, high energy density, and high stability, and is applicable to future flexible electronic device.

In conclusion, in the quinone compound-graphene composite material provided by the first aspect of the embodiments of the present invention, high conductivity graphene and the quinone compound are chemical compounded. The composite material has high energy density, high flexibility, high conductivity, and high stability, which solves the problem in the prior art that organic cathode material has poor conductivity and poor stability. The composite material can be used as cathode material for preparing flexible battery. The quinone compound-graphene composite material preparation methods provided by the second and third aspects of the embodiments of the present invention have simple process, and are easy to implement in industrialized production. The flexible lithium secondary battery provided by the fourth aspect of the embodiments of the present invention exhibits high flexibility, high rate performance, high energy density, and high stability, and is applicable to future flexible electronic device.

### Embodiment 1

A preparation method of quinone compound-graphene composite material includes the following steps:

In step S11, aminate p-benzoquinone: Use nitration reduction method. Specifically, nitrate the p-benzoquinone by mixed acid containing concentrated sulfuric acid and concentrated nitric acid, to obtain dinitro p-benzoquinone; add sodium sulfide to perform reduction, to obtain mixed liquor containing the aminated p-benzoquinone. Filter, wash, and dry the mixed liquor, to obtain aminated p-benzoquinone powder, where a reaction formula of the amination reaction is shown in formula (1):

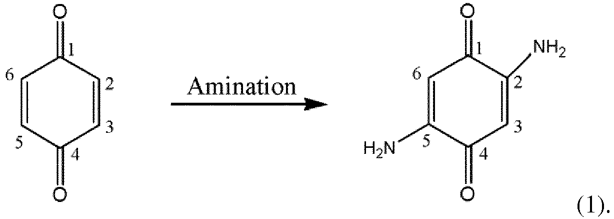

In step S 12, carboxylate graphene: Add, to graphene oxide powder, mixed acid that contains concentrated sulfuric acid and concentrated nitric acid in the volume ratio of 3:1, to react for 12 h at 60 °C under conditions of stirring and ultrasonic dispersion; and perform filtration by filter membrane, washing by distilled water, and vacuum drying, to obtain carboxylated graphene powder with surface connected to large amount of carboxyl, where the chemical structural formula of the carboxylated graphene is shown in formula (2):

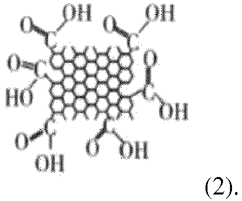

In step S13, perform amidated compounding: Add the excessive aminated p-benzoquinone powder obtained in step S11 and the carboxylated graphene powder obtained in step S12 to water; expose them to ultrasound for 2 h, to obtain mixed dispersed liquor; and perform reflux on the mixed dispersed liquor for 72 h at 120 °C under protection of nitrogen gas N₂, to perform amidation reaction (as shown in formula (3)):

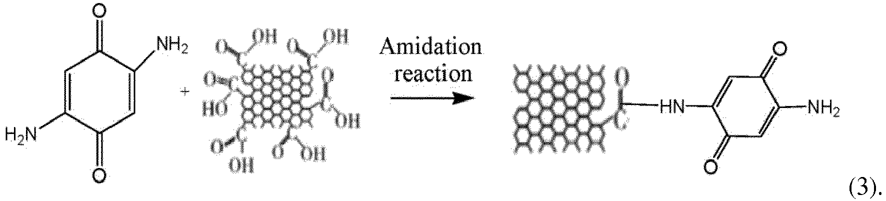

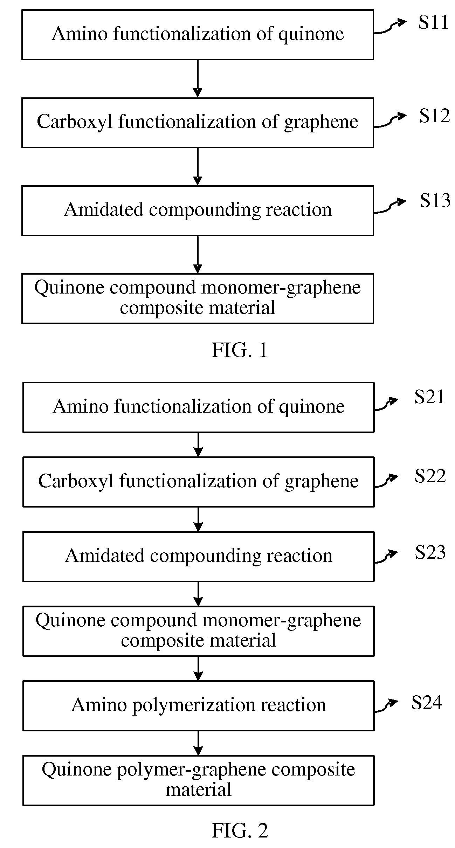

After the reaction is finished, perform filtration on the product by filter membrane, washing, and vacuum drying at 60 °C, to obtain graphene composite material with surface connected to p-aminated benzoquinone group, that is, p-benzoquinone-graphene composite material. FIG. 1 is flowchart of preparing the p-benzoquinone-graphene composite material according to this embodiment.

### Embodiment 2

A preparation method of quinone compound-graphene composite material includes the following steps:
In step S21, aminate 1,5-anthraquinone: Use nitration reduction method. Specifically, nitrate the 1,5-anthraquinone by mixed acid containing concentrated sulfuric acid and concentrated nitric acid, to obtain 1,5-dinitro anthraquinone; add sodium sulfide to perform reduction, to obtain mixed liquor containing 1,5-diaminated anthraquinone. Filter, wash, and dry the mixed liquor, to obtain 1,5-diaminated anthraquinone powder, where the amination reaction formula is shown in formula (4): 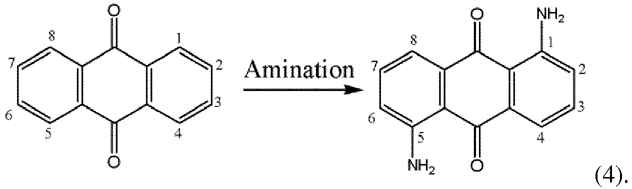
In step S22, carboxylate graphene: Add, to graphene oxide powder, mixed acid that contains concentrated sulfuric acid and concentrated nitric acid in the volume ratio of 3:1, to react for 12 h at 50 °C under conditions of stirring and ultrasonic dispersion; and perform filtration by a filter membrane, perform washing by distilled water, and perform vacuum drying, to obtain carboxylated graphene powder with a surface connected to a large amount of carboxyl.
In step S23, perform amidated compounding: Add the excessive 1,5-diaminated anthraquinone powder obtained in step S21 and the carboxylated graphene powder obtained in step S22 to water; expose them to ultrasound for 2 h, to obtain mixed dispersed liquor; and perform reflux on the mixed dispersed liquor for 72 h at 120 °C under the protection of nitrogen gas N₂, to perform amidation reaction (as shown in formula (5)):

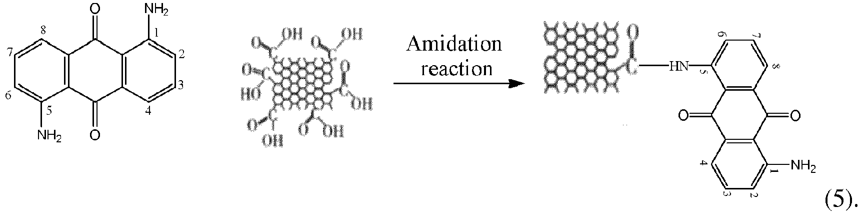

After the reaction is finished, perform filtration on the product byfilter membrane, washing, and vacuum drying at 60 °C, to obtain graphene composite material with surface connected to p-aminated anthraquinone group, that is, aminated anthraquinone-graphene composite material.

In step S24, polymerize amino: Weigh out quantitative aminated anthraquinone-graphene, perchloric acid (1 mol/L), hydrogen peroxide (saturated water solution), and potassium dichromate according to molar ratio of 1:2:2:2, and add them to acetonitrile solvent; after mixing them well, add saturated water solution of potassium dichromate slowly and dropwise, and stir them for reaction for 72 h at 35 °C under protection of argon gas; and then perform decompression filtration, washing by deionized water, and vacuum drying for 24 h at 80 °C, to obtain solid powder, that is, polyaminated anthraquinone-graphene composite material. A reaction formula of the amino polymerization reaction is shown in formula (6):

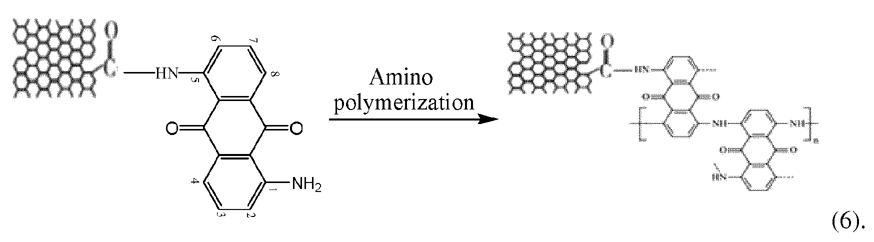

FIG. 2 is flowchart of preparing the polyaminated anthraquinone-graphene composite material according to this embodiment.

### Embodiment 3

A preparation method of quinone compound-graphene composite material includes the following steps:

In step S31, aminate 1,5-anthraquinone: Use nitration reduction method. Specifically, nitrate the 1,5-anthraquinone by mixed acid in concentrated sulfuric acid, to obtain 1,5-dinitro anthraquinone; add sodium sulfide to perform reduction, to obtain mixed liquor containing 1,5-diaminated anthraquinone. Filter, wash, and dry the mixed liquor, to obtain 1,5-diaminated anthraquinone powder.

In step S32, polymerize amino: Add the 1,5-diaminated anthraquinone powder to electrolyte of organic propylene carbonate solvent, and perform polymerization reaction by constant potential polymerization method of three-electrode system, where the polymerization reaction mainly happens in amino connected to benzene ring and hydrogen in para-position of the amino in monomer, and the amino connected to the benzene ring and the hydrogen in para-position of the amino form poly-1,5-diamino anthraquinone macromolecular chain, that is, the 1,5-diaminated anthraquinone, mainly in head-to-tail ligation manner, where a reaction formula of the amino polymerization reaction is shown in formula (7):

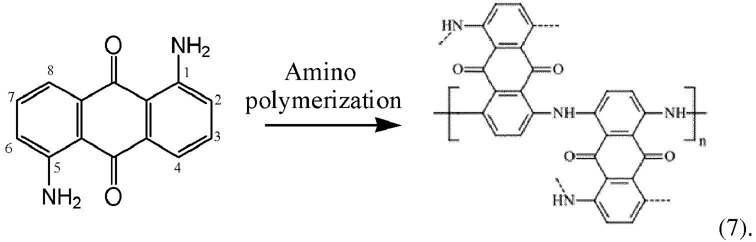

In step S33, carboxylate graphene: Add, to graphene oxide powder, mixed acid that contains concentrated sulfuric acid and concentrated nitric acid in the volume ratio of 3:1, to react for 12 h at 60 °C under conditions of stirring and ultrasonic dispersion; and perform filtration by filter membrane, washing by distilled water, and vacuum drying, to obtain carboxylated graphene powder with surface connected to large amount of carboxyl.

In step S34, perform amidated compounding: Add the excessive 1,5-diaminated anthraquinone powder obtained in step S31 and the carboxylated graphene powder obtained in step S33 to water; expose them to ultrasound for 2 h, to obtain mixed dispersed liquor; and perform reflux on the mixed dispersed liquor for 72 h at 120 °C under protection of nitrogen gas N₂, to perform amidation reaction (as shown in formula (8)):

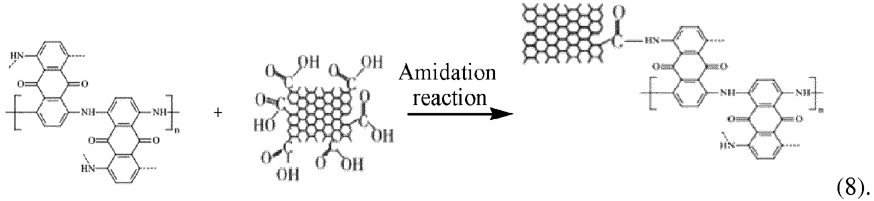

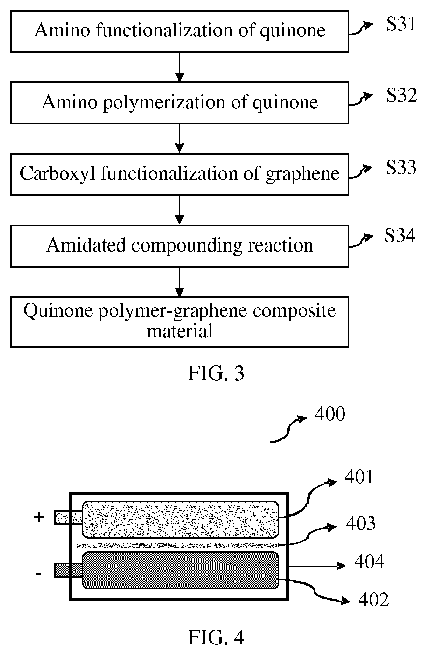

After the reaction is finished, perform filtration on the product by filter membrane, washing, and vacuum drying at 60 °C, to obtain graphene composite material with surface connected to p-aminated anthraquinone group, that is, polyaminated anthraquinone-graphene composite material. FIG. 3 is flowchart of preparing the polyaminated anthraquinone-graphene composite material according to this embodiment.

### Embodiment 4

A schematic structural diagram of a flexible lithium secondary battery is shown in FIG. 4. The flexible lithium secondary battery (400) includes flexible battery housing (404); flexible cathode(401), flexible anode (402), and separator (403) that are located inside the flexible battery housing (404), and electrolyte injected inside the flexible battery housing (404), where the separator (403) is disposed between the flexible cathode (401) and the flexible anode (402). The p-benzoquinone-graphene composite material obtained in Embodiment 1 is used as cathode active material of the flexible cathode (401), and graphene is used as anode active material of the flexible anode (402).

Cathode electrode preparation: mix the cathode active material, which is the p-benzoquinone-graphene composite material, with polyvinylidene fluoride which serves as binder and with carbon nanotube which serves as conductive agent; add certain amount of organic solvents N-methyl pyrrolidone, to obtain cathode slurry; and evenly coat onto aluminum foil current collector with the obtained cathode slurry, and perform baking and tablet compression to obtain the cathode electrode.

Anode electrode preparation: mix the anode active material graphene with polyvinylidene fluoride which serves as binder and with carbon nanotube which serves as conductive agent; add certain amount of organic solvents N-methyl pyrrolidone, to obtain anode slurry; and evenly coat onto copper foil current collector with the obtained anode slurry, and perform baking and tablet compression to obtain the anode electrode.

The obtained cathode electrode, PP/PE/PP polyolefin separator, and the obtained anode electrode are stacked in sequence and assembled, and after an electrolyte is added, packaging is performed by the flexible battery housing, to obtain the flexible lithium secondary battery which cathode active material is the quinone compound-graphene composite material.

### Embodiment 5

A flexible lithium secondary battery is provided, where the flexible lithium secondary battery uses the polyaminated anthraquinone-graphene composite material, which is obtained in Embodiment 2, as cathode active material, and is assembled by the method which is the same as the method in Embodiment 4.

### Embodiment 6

A flexible lithium secondary battery is provided, where the flexible lithium secondary battery uses the polyaminated anthraquinone-graphene composite material, which is obtained in Embodiment 3, as cathode active material, and is assembled by the method which is the same as the method in Embodiment 4.

### Comparative example 1

A flexible lithium secondary battery is provided, where the flexible lithium secondary battery uses p-benzoquinone as cathode active material and is assembled by the method which is the same as the method in Embodiment 4.

### Comparative example 2

A flexible lithium secondary battery is provided, where the flexible lithium secondary battery uses polyaminated anthraquinone as cathode active material, and is assembled by the method which is the same as the method in Embodiment 4.

Results embodiment: To support beneficial effects of the embodiments of the present invention, the effect embodiment is provided below, evaluating the performance of product provided by the embodiments of the present invention.

Tests of charge-discharge cycles and rate performance are performed bybattery performance tester on the flexible lithium secondary batteries obtained in Embodiment 4 to Embodiment 6 and comparative example 1 and comparative example 2. For the cycle test, voltage range is 1.5 to 3.6 V, and current is 0.2 C; and after 50 cycles, battery capacity is recorded and capacity retention ratio is calculated. For the rate test, voltage range is 1.5 to 3.6 V, and current is 5.0 C; and discharging capacity is recorded and rate discharging ratio is calculated.

To further test the battery flexibility, the flexible lithium secondary batteries are bended repeatedly for 10 times in the horizontal direction and vertical direction at bending angle of less than or equal to 10 degree, and then the tests of charge-discharge cycles and rate performance are performed, where test conditions are the same as the above. Test results are shown in Table 1:

**Table 1: Performance comparison of flexible lithium secondary batteries in the embodiments and comparative examples**

| | Cathode discharging capacity (mAh/g) | Rate discharging ratio at 5.0C(%) | Cyclic capacity retention ratio at 0.2C(%) | Rate discharging ratio at 5.0C after bending(%) | Cyclic capacity retention ratio at 0.2C after bending(%) |
|---|---|---|---|---|---|
| Embodiment 4 | 155.3 | 80.6% | 79.1% | 68.4% | 71.4% |
| Embodiment 5 | 231.2 | 84.1% | 78.7% | 70.3% | 65.6% |
| Embodiment 6 | 254.6 | 86.3% | 84.2% | 71.8% | 72.6% |
| Comparative example 1 | 124.1 | 54.2% | 60.2% | 32.4% | 38.4% |
| Comparative example 2 | 201.8 | 45.8% | 57.3% | 28.7% | 40.2% |

The results in Table 1 indicate that: the flexible lithium secondary batteries provided by the embodiments of the present invention deliver good rate performance and cyclic performance, and can continue to maintain good rate performance and cyclic performance after being bended. This is achieved because the flexible lithium secondary batteries provided by the embodiments of the present invention use the quinone compound-graphene composite material as the cathode active material, and the composite material is compounded of high-conductivity graphene and quinone compound, and thereby exhibits high energy density, high flexibility, high conductivity, and high stability, and is well applicable to preparation of flexible battery.

## Claims

1. A quinone compound-graphene composite material, comprising quinone compound and graphene, wherein the quinone compound is chemically bonded on the surface of the graphene, and the quinone compound is quinone compound monomer or quinone polymer.

2. The quinone compound-graphene composite material according to claim 1, wherein the surface of the graphene contains carboxyl functional group, the quinone compound contains amino functional group, and the quinone compound is bonded on the surface of the graphene by amido bond formed by the amino functional group and the carboxyl functional group.

3. The quinone compound-graphene composite material according to claim 1 or 2, wherein the quinone compound monomer is any one of benzoquinone, naphthoquinone, anthraquinone, and phenanthraquinone, or the isomer of any one of benzoquinone, naphthoquinone, anthraquinone, and phenanthraquinone.

4. The quinone compound-graphene composite material according to claim 1 or 2, wherein the quinone polymer is the polymer formed by any one or more than one of benzoquinone, naphthoquinone, anthraquinone, and phenanthraquinone, or the polymer formed by the isomer of any one of benzoquinone, naphthoquinone, anthraquinone, and phenanthraquinone or formed by isomers of more than one of benzoquinone, naphthoquinone, anthraquinone, and phenanthraquinone.

5. The quinone compound-graphene composite material according to claim 1 or 2, wherein the quinone compound accounts for 20 to 80% by weight of the quinone compound-graphene composite material.

6. A preparation method of quinone compound-graphene composite material, comprising the following steps:
aminating quinone compound monomer by nitration reduction method, to obtain mixed liquor containing the aminated quinone compound monomer;
filtering, washing, and drying the mixed liquor containing the aminated quinone compound monomer, to obtain aminated quinone compound monomer powder;
adding graphene oxide powder to mixed acid that contains concentrated sulfuric acid and concentrated nitric acid in preset proportion, to react for 5 to 15 h at 50 to 60 °C, and then performing operations of filtration, washing, and vacuum drying, to obtain surface-carboxylated graphene powder;
adding the aminated quinone compound monomer powder and the surface-carboxylated graphene powder to water or ethyl alcohol, and performing ultrasonic dispersion to obtain mixed dispersed liquor; and
performing reflux reaction on the mixed dispersed liquor for 48 to 72 h at 100 to 120 °C under protection of nitrogen gas, and after reaction is finished, performing filtration, washing, and drying on the product, to obtain the quinone compound monomer-graphene composite material.

7. The quinone compound-graphene composite material preparation method according to claim 6, wherein the quinone compound monomer is any one of benzoquinone, naphthoquinone, anthraquinone, and phenanthraquinone, or the isomer of any one of benzoquinone, naphthoquinone, anthraquinone, and phenanthraquinone.

8. The quinone compound-graphene composite material preparation method according to claim 6 or 7, wherein the reaction for 5 to 15 h at 50 to 60 °C is performed under conditions of stirring and ultrasonic dispersion.

9. The preparation method of quinone compound-graphene composite material according to claim 6 or 7, further comprising the following steps:
weighing out the quinone compound monomer-graphene composite material, perchloric acid, saturated water solution of hydrogen peroxide, and potassium dichromate according to molar ratio of 1:2:2:2, and adding them to acetonitrile solvent, to obtain mixed solution;
adding saturated water solution of potassium dichromate dropwise to the mixed solution to react for 48 to 72 h at 30 to 50 °C under protection of argon gas, to obtain mixed liquor containing quinone polymer-graphene composite material; and
performing decompression filtration, washing, and vacuum drying on the mixed liquor containing the quinone polymer-graphene composite material, to obtain the quinone polymer-graphene composite material.

10. A preparation method of quinone compound-graphene composite material, comprising the following steps:
aminating quinone compound monomer by nitration reduction method, to obtain mixed liquor containing the aminated quinone compound monomer;
filtering, washing, and drying the mixed liquor containing the aminated quinone compound monomer, to obtain aminated quinone compound monomer powder;
adding the aminated quinone compound monomer powder to electrolyte of organic propylene carbonate solvent, and polymerizing the aminated quinone compound monomer by constant potential polymerization method of three-electrode system, to obtain animated quinone polymer;
adding graphene oxide powder to mixed acid that contains concentrated sulfuric acid and concentrated nitric acid in preset proportion, to react for 5 to 15 h at 50 to 60 °C, and then performing operations of filtration, washing, and vacuum drying, to obtain surface-carboxylated graphene powder;
adding the aminated quinone polymer and the carboxylated graphene powder to water or ethyl alcohol, and performing ultrasonic dispersion to obtain mixed dispersed liquor; and
performing reflux reaction on the mixed dispersed liquor for 48 to 72 h at 100 to 120 °C under the protection of nitrogen gas, and after the reaction is finished, performing filtration, washing, and drying on the product, to obtain quinone polymer-graphene composite material.

11. The quinone compound-graphene composite material preparation method according to claim 10, wherein the quinone compound monomer is any one of benzoquinone, naphthoquinone, anthraquinone, and phenanthraquinone, or the isomer of any one of benzoquinone, naphthoquinone, anthraquinone, and phenanthraquinone.

12. The quinone compound-graphene composite material preparation method according to claim 10 or 11, wherein the reaction for 5 to 15 h at 50 to 60°C is performed under conditions of stirring and ultrasonic dispersion.

13. A flexible lithium secondary battery, comprising flexible battery housing; flexible cathode, flexible anode, and separator that are located inside the flexible battery housing, and electrolyte injected inside the flexible battery housing, where the separator is disposed between the flexible cathode and the flexible anode, the flexible cathode includes cathode active material which including quinine-compound graphene composite material. The quinone compound-graphene composite material includes quinone compound and graphene, where the quinone compound is chemically bonded on the surface of the graphene, and the quinone compound is quinone compound monomer or quinone polymer.

14. The flexible lithium secondary battery according to claim 13, wherein the flexible anode comprises anode active material, and the anode active material is graphene, carbon nanotube, silicon film, or tin film.
